# EUROPEAN PATENT APPLICATION

(11) **EP 2 272 559 A1**
(43) Date of publication of application: **12.01.2011**
(21) Application number: 09164041.7
(22) Date of filing: 29.06.2009
(51) Int. Cl.: A61M 39/02, A61M 5/158

(54) **Infusion Device**

(71) Applicant: Unomedical A/S, 3460 Birkerød (DK)
(72) Inventor: Gyrn, Steffen, 4100, Ringsted (DK); Thorup, Sören, 2700, Brønshøj (DK); Dirac, Holger, 3460, Birkerød (DK)
(74) Representative: Nilausen, Kim

(57) **Abstract**

The invention concerns a medical device which is inserted into the subcutaneous or intramuscular area of a patient which needs medication or measurement of parameters obtained from subcutaneously obtainable bodily fluids.

A medical device according to the invention is constituted by a site (1) and a connector part (2) where the site (1) is stationary during use and attached to a patients skin and the connector part (2) can be attached to the site (1) during use which site (1) comprises
- a surface material (3) to be attached to the patients skin;
- an inlet for fluid and a fluid path leading the fluid to a subcutaneous position;
- positioning means (5) for the connector part (2) configured to provide a stationary i.e. fixed positioning of the connector part (2) relative to the site (1) in a horizontal direction during use; and
which connector part (2) comprises
- an outlet for fluid (18) which during use protrudes through the inlet for fluid of the site (1);
- means for positioning (7) corresponding to the positioning means (5) on the site (1).

The medical device is a two piece device in the use-situation and the site (1) is provided with positioning means (5) defining more than one possible position for the connector part (2) during use.

## Description

### Technical field of the invention

The invention concerns a medical device which is inserted into the subcutaneous or intramuscular area of a patient which needs medication or measurement of parameters obtained from subcutaneously obtainable bodily fluids.

### Background of the invention

Conventional infusion devices comprising two parts a base with a cannula, and a corresponding connector part rely on accurate placement of the base, as the base and the connector only fit together in one relative position to each other. Often, the base part is not symmetrical, and allows for only one position of connector part including tubing or other connections. Thus once the base is attached and/or the cannula is inserted, the relative position of the infusion device is determined, and e.g. position of a tubing or the like cannot be changed without inserting a new device. Misplacing the base part would increase expenses and cause discomfort to the patient.

Another issue related to cannula-based medical devices is the risk of leakage and/or blockage during use. For patients depending on accurate medical doses, such as diabetes patients depending on accurate dosage insulin, leakage or blockage of the delivery device can cause a health-, or even life-threatening situation for the patient, when delivering e.g. less insulin than anticipated.

Thus, there is an obvious need in the art for a robust, reliable, accurate, safe, hygienic, and user friendly insertion device, which addresses the issues discussed above.

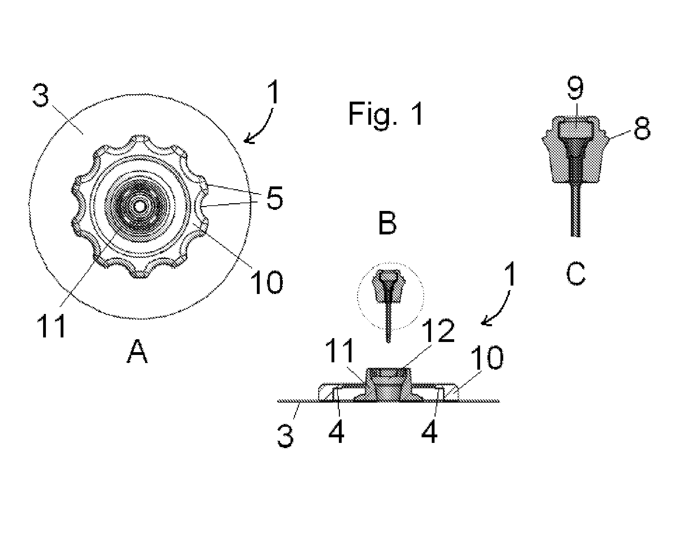

WO 2005/046780 illustrates an infusion device of the same type as the medical device according to the present invention i.e. an infusion device comprising a site to be placed on the skin of a patient which site comprises a subcutaneously positioned cannula, and which site during transfer of liquid is connected to a connector part comprising a connector needle. Also it is possible to manipulate the site so that the connector part can be attached to the site at different distinct positions. Contrary to the present invention, the site though is constituted by two separate parts (100 (fig. 1),250 (fig. 27-31)) which has to be attached to each other before the connector part (200 (fig. 33-34) can be placed in the desired position. This device is a three piece device in a use-situation as the user has to handle two separate parts together with the infusion site already placed on the patients skin.

The present invention is a two piece device in the use-situation as the user only has to handle one part together with the infusion site placed on the patients skin.

### Summary of the invention

The object of the invention is to provide medical device comprising a site (1) and a connector part (2) where the site (1) is stationary during use and attached to a patients skin and the connector part (2) can be attached to the site (1) during use which site (1) comprises
- a surface material (3) to be attached to the patients skin;
- an inlet for fluid and a fluid path leading the fluid to a subcutaneous position;
- positioning means (5) for the connector part (2) configured to provide a stationary i.e. fixed positioning of the connector part (2) relative to the site (1) in a horizontal direction during use; and
   which connector part (2) comprises
- an outlet for fluid (18) which during use protrudes through the inlet for fluid of the site (1);
- means for positioning (7) corresponding to the positioning means (5) on the site (1) wherein the medical device is a two piece device in the use-situation and the site (1) is provided with positioning means (5) defining more than one possible position for the connector part (2) during use.

According to one embodiment the connector part (2) is releasably attached to the site (1) and the site (1) comprises attachment means (4) for the connector part (2) providing releasable attachment of the connector part (2) to the site (1) during use; and the connector part (2) comprises corresponding means for attachment (6) configured to interact with the attachment means (4) on the site (1).

According to one embodiment the positioning means (5) of the site (1) comprise at least two openings (5) where at least one opening (5) correspond(s) to a protruding part (7) on the connector part (2) when the connector part (2) is placed in a use position. The positioning means (5) of the site (1) can comprise at least two openings (5) where two or more openings (5) correspond to two or more protruding part (7) on the connector part (2) when the connector part (2) is placed in a use position.

According to one embodiment the positioning means (5) of the site (1) comprise 2 to 10 openings (5) corresponding to 2 to 10 protruding parts (7) on the connector part (2) when the connector part (2) is placed in a use position.

According to one embodiment the surface material (3) to be attached to the patient's skin is adhesive on at least one surface.

According to one embodiment the site (1) has a subcutaneous part when positioned on the patients skin or has means for attaching a subcutaneous part after positioning the site on the patients skin. The subcutaneous part can be a soft or hard cannula or a sensor or a combination of a cannula and a sensor.

According to one embodiment the attachment means (4) of the site (1) comprises an increase or a decrease in a cross-sectional dimension providing an inward or outward step in a vertical profile. The attachment means can be provided as an outward rim at an outer vertical surface or an inward rim at an inner vertical surface.

According to one embodiment the means for attachment (6) of the connector part (2) comprises actuation means (15) in the form of at least one arm placed along the outer edge of the connector part (2) which arm is fastened to the connector part (2) at one end and can be pivoted around this fastening point, the arm is provided with retaining means (14) configured to prevent vertical movements of the connector part (2) relative to the site (1).

According to one embodiment the means for attachment (6) of the connector part (2) comprises actuation means (15) in the form of two oppositely positioned arms which arms each are pivotally attached at one end and each arm is provided with retaining means (14) and the distance between the retaining means on each arm can be varied due to the pivotal movement of the two arms.

According to one embodiment the connector part (2) comprises means for transferring liquid from the connector part (2) to the patient.

According to one embodiment the connector part (2) comprises a connector needle in the form of a tubular element (18) to be inserted through a septum of the site (1). The connector part (2) can be provided with guiding means (17) adapted to guide the tubular element (18) through a penetratable part of the site (1) in a vertical direction.

According to one embodiment the site (1) during use comprises a septum facing upwards i.e. having an open surface in a vertical direction. Normally the septum has the form of a flat or disc-shaped portion having same cross-section as the cavity surrounding it.

According to one embodiment the device comprises a leakage indication system comprising leakage indication means adapted to provide a colour change upon leakage of a fluid, the indication means being provided on a surface of the site (1) near the insertion site, and being visible i.e. inspectable upon use. The leakage indication means can be insulin detection means.

According to one embodiment the leakage indication system comprises a reference marker indicating the visual change to be expected upon leakage. The visible change could e.g. be a change of colour of the indicator means.

According to one embodiment the device comprises a subcutaneous part comprising a coloured or contrast-enhanced cannula, and the site (1) comprises a transparent area arranged near the insertion site, said transparent area being adapted to allow visible detection of misplacement of the cannula during use.

### Definitions

"Proximal" means defines a part of or a side of a unit which compared to a similar opposite part or side of the same unit is closest to the patients skin when the unit is in use.

"Distal" defines the part or side of a unit which is opposite the proximal part or side of the same unit.

When there is referred to that something is "vertical" then there is referred to a line or direction which would be perpendicular to the patients skin when the device is in use.

When there is referred to that something is "horizontal" then there is referred to a line or direction which would be parallel to the patients skin when the device is in use. Also, a rotation movement in a plane level parallel to the patients skin is considered to be a "horizontal movement".

### Brief description of the drawings

A detailed description of embodiments of the current invention will be made with reference to the accompanying figures, wherein like numerals designate corresponding parts in different figures.
Figs. 1A-C show a site of a first embodiment of a medical device according to the invention: A: the site seen from above; B: the site and a cannula part seen from the side; C: the cannula part seen from the side.

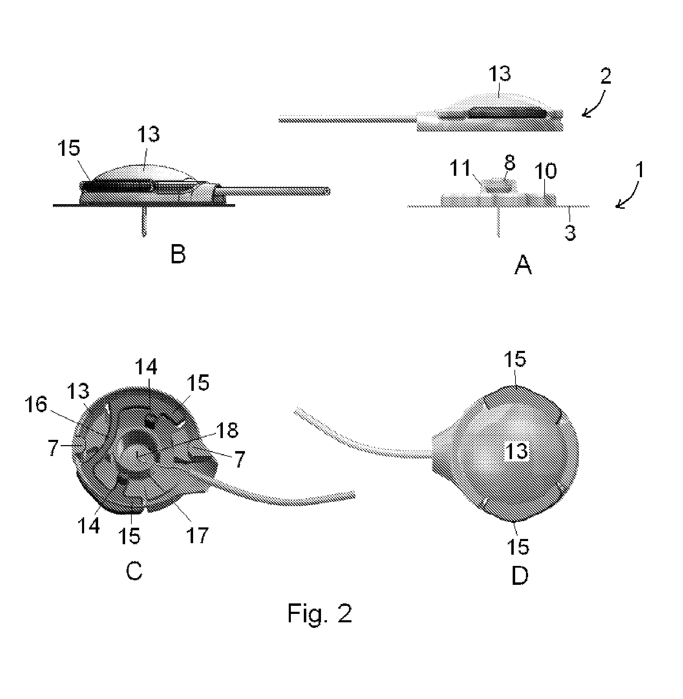

Figs. 2A-D show the site and a connector part according to a first embodiment of the medical device: A: the connector part and the site provided with a cannula part in position just before assembling; B: the connector part and the site shown in A in assembled position; C: connector part seen from below; D: Connector part seen from above.

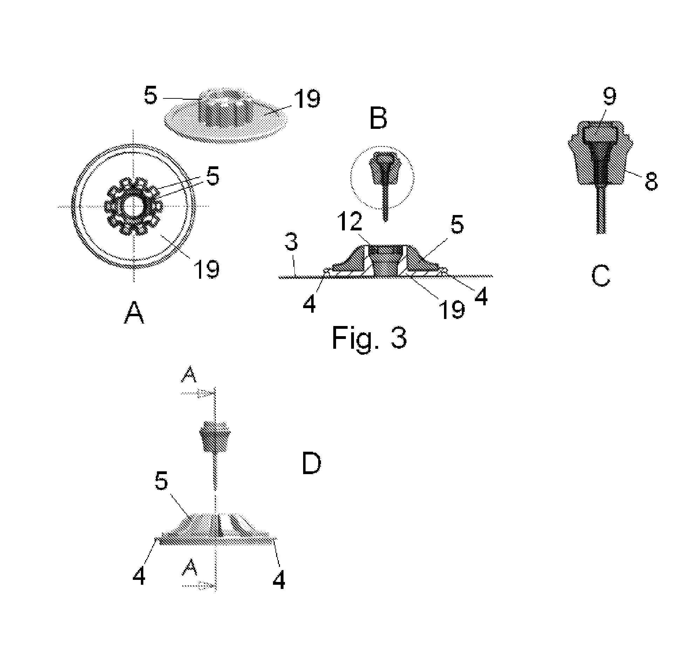

Fig. 3A-D show a second and a third embodiment of a site of a medical device according to the invention: A: a second embodiment of a site seen from above and from a side view; B: the site of A and a cannula part seen from the side; C: the cannula part seen from the side; D: a third embodiment of a site seen from the side.

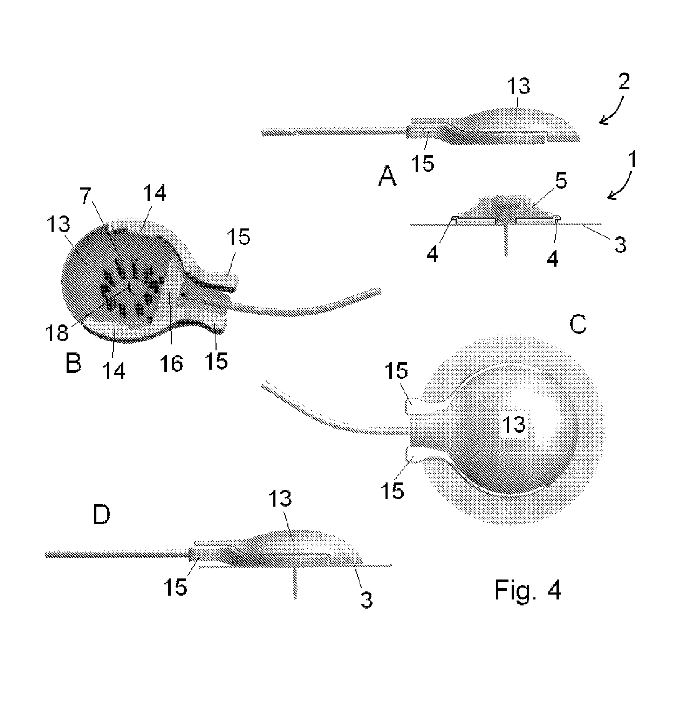

Fig. 4 shows show the site and a connector part according to a second or third embodiment of the medical device: A: the connector part and the site provided with a cannula part in position just before assembling; B: the connector part and the site shown in A in assembled position; C: connector part seen from below; D: Connector part seen from above.

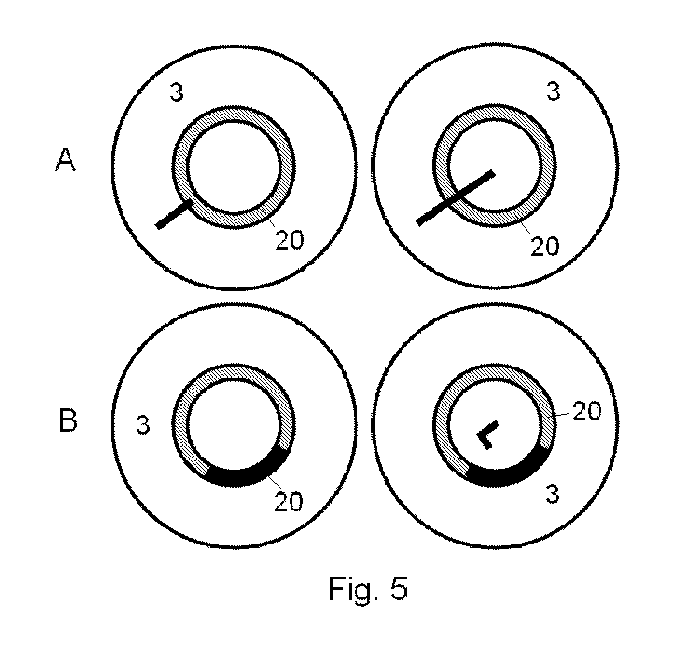

Fig. 5 shows a leakage indication system. Left: top view; right: bottom view. A: example of a "complete crawl out" where a portion of the catheter or cannula becomes visible through a transparent plaster. B: example of a "partial crawl out" or "buckling/crimping" of the catheter or cannula, where the catheter or cannula is not visible through the transparent plaster.

### Detailed description of the invention

Fig. 1A, B and C shows a first embodiment of a medial device according to the invention, the medical device is a device comprising a site 1 such as an infusion site which can be combined with a subcutaneous part 8 such as a cannula part. If the subcutaneous part is a cannula part 8, it comprises a disc like septum 9 blocking the entrance to a cannula which cannula during use is positioned subcutaneously. The site 1 comprises three parts: a surface material 3 which during use is attached to the patients skin, a circular or ring-shaped part 10 attached unreleasably to the upper surface of the surface material 3 and a central part 11 which is also attached unreleasably to the upper surface of the surface material 3 and which part is adapted to hold the subcutaneous part 8. The surface material 3 is normally made of a patch of weaved or non-weaved material and is adapted to be releasably attached to the patient's skin during use, e.g. by an adhesive surface facing the skin. The site 1 comprises positioning means 5 corresponding to positioning means 7 on a connector part, such a connector part can have the form illustrated in fig. 2. According to the first embodiment, the positioning means 5 are part of the circular part 10 and are placed along the periphery of the circular part 10.

The central part 11 comprises an opening 12 or cavity, adapted to accommodate a portion of a subcutaneous part 8, essentially the portion of the subcutaneous part 8 which is not inserted or to be inserted in the patient's skin. The opening 12 may comprise attachment means for the subcutaneous part 8, adapted to provide a non-releasable connection during use and the subcutaneous part 8 comprises corresponding means for attachment to the opening 12 and thereby to the infusion site 1. An embodiment of a subcutaneous part according to the invention is shown in fig. 1C.

The positioning means 5 generally comprises two or more openings or recesses in combination with protruding parts provided in a hard part of the infusion site 1; hard parts of the site are e.g. the circular part 10 as in the first embodiment or e.g. the central part although placement of the positioning means in this part is not illustrated by an embodiment.

The positioning means 5 of the first embodiment are shaped like a symmetric toothed wheel having 10 teeth with rounded off openings or recesses provided in between the teeth. Each opening or recess has identical dimensions, and a given area comprising both an opening and a surrounding part of a protruding area fits closely to a corresponding area on the proximal side of the connector part 2. The corresponding means for positioning 7 of the connector part 2 are adapted to fit into one or more of the openings of the positioning means 5, whereby ten distinct and different relative positions of the connector part 2 in correspondence to the site 1 are possible. The toothed wheel will normally have 3-20 openings, thereby providing 3-20 different positions of the connector part 2 relative to the site 1.

The openings of the toothed wheel might have less rounded shape comprising straighter edges, such as triangular or rectangular openings. Accordingly, the means for positioning 7 will have a corresponding shape, such as a triangular or rectangular shape, respectively.

The toothed wheel and the central part 11 can be attached to or be part of a base of a hard material i.e. the base, the circular part 10 comprising the toothed wheel and the central part 11 comprising the subcutaneously positioned part are moulded as one single piece which is afterwards attached to a surface material 3 having a non-adhesive upper or distal side and an adhesive lower or proximal side, or alternatively, the toothed wheel and the central part 11 can be provided individually and each part is then attached directly to a surface material having a non-adhesive upper or distal side and an adhesive lower or proximal side.

In order to provide a reliable and releasable attachment of the connector part 2 to the site 1 during use, the site 1 comprises attachment means 4. According to the first embodiment the attachment means 4 are provided on the circular part 10 which circular part 10 is essentially shaped like a hollow cylinder having upright walls essentially perpendicular to the surface material 3. The profile of the inner surface of the upright wall is shaped as a reverse L i.e. an upside down L: Γ. This shape makes it possible for a part of the connector part 2 to hook around the profile. The same effect of providing an edge, around which a part of the connector part 2 can get hooked, can be provided if the inner surface of the upright wall is e.g. provided with an annular groove at the upper or distal part of the wall. The attachment means 4 are provided by a section of inner profile having a smaller diameter near the top.

Fig. 2 shows a first embodiment of a connector part 2 according to the invention comprising a hard outer shell 13 having an inner cavity, adapted to encompass the site 1. The outer diameter of the connector part 2 is normally slightly wider than the site 1, but might be similar to or smaller than the outer diameter of the site 1. Furthermore, the connector part 2 comprises means for attachment 6 corresponding to attachment means 4 of the site 1 and means for positioning 7 corresponding to positioning means 5 of the site 1. When the corresponding attachment means 6 are activated, the connector part 2 can be attached releasably to the site 1 as the means for attachment 6 correspond to and/or interact with the attachment means 4 of the site 1. The means for positioning 7 corresponding to the positioning means 5 will - when the connector part 2 is attached to the site 1 - provide that the connector part 2 is positioned in a distinct user-defined position relative to the site 1. When combined, the means for positioning 7 of the connector part 2 and the positioning means 5 of the site 1 are adapted to define more than one distinct position for the connector part 2 in relation to the site 1 which positions the user can choose among when the user joins the two parts are together.

The means for attachment 6 of the connector part 2 comprise actuating means 15, retaining elements 14 and an elastic element 16. The actuating means 15 comprises two arms positioned diametrically opposite each other and each arm is provided with an outward hook constituting a retaining element 14. Each hook has a portion which upon release is caught under the protruding upper edge of the central opening and prevents the connector part 2 from moving away in a vertical direction. The two arms of the actuating means 15 form part of the outer periphery of the connector part 2 and will normally form a section with an increase diameter or cross-section which will make it possible for the user to feel exactly where to push in order to release/attach the connector part 2 from the site 1. The elastic element 16 connects the two arms by one end of each arm. When the two arms forming the actuating means 15 are pressed towards each other, the elastic element will provide a spring action trying to return the arms to their original relaxed position. The three elements: actuating means 15, retaining elements 14 and elastic element 16 could be moulded as a single element.

By applying an inward directed pressure on the actuating means 15, e.g. by pressing e.g. with two fingers, the distance between the retaining elements14 is reduced to less than the maximum diameter of the top section of the circular part 10, whereupon the connector part 2 can be attached to or removed from the site 1 as the retaining elements 14 can then pass through the opening provided by the top section of the circular part 10. Upon release of the inward pressure and assisted by the action of the elastic element 16, the retaining elements 14 move back into or towards the position, where the distance between the retaining elements 14 is increased to a distance greater than diameter of the top section of the circular part 10.

The connector part 2 comprises a central tubular element 18 through which a fluid can be delivered. The tubular element 18 is encompassed by a cylindrical wall 17 standing upright from the inner surface of the outer shell, the cylindrical wall 17 is provided with an opening through which a pipeline for fluid can pass. The cylindrical wall 17 provide guidance for the user in order to determine the correct position of the connector part 2 relative to the site 1 when the user pushes the connector part 2 towards the site 1.

The corresponding means for positioning 7 are two protrusions attached to or being part of the outer shell 13 and facing inwards toward the centre of the connector part 2. The two protrusions are according to this embodiment positioned symmetrically opposite each other. Alternatively, the connector element can have an asymmetric distribution of protrusions and the connector element can also have only one, or more than two protrusions e.g. up to 10 protrusions. The protrusions could according to the invention be placed in any angle if the design of the actuating means 15 allows it. As mentioned above, the shape and position of the protrusion(s) are adapted to the positioning means 5 of the site 1.

Fig. 3A shows a second embodiment of a medial device according to the invention, the medical device is as the embodiment illustrated in fig. 1 a device comprising a site 1 which can be combined with a subcutaneous part 8. If the subcutaneous part 8 is a cannula part, it comprises a disc like septum 9 blocking the entrance to the cannula which during use is positioned subcutaneously. The site 1 comprises two parts: a surface material 3 which during use is attached to the patients skin and a top part 19 which is attached unreleasably to the upper surface of the surface material 3 and which part is adapted to hold the subcutaneous part 8 and provide for the releasable attachment of a connector part 2. The surface material 3 is normally made of a patch of weaved or non-weaved material and is adapted to be releasably attached to the patient's skin during use, e.g. by an adhesive surface facing the skin. The site 1 comprises positioning means 5 corresponding to positioning means 7 on a connector part, such a connector part 2 can have the form illustrated in fig. 4. According to the second embodiment, the positioning means 5 are part of the top part 19 and are placed along the periphery of a central part of the top part 19.

This second embodiment also provides positioning means 5 similar to a toothed wheel when seen from the top. The toothed wheel has teeth and openings of a more rectangular shaped than the first embodiment and the vertical profile of the outer periphery of the toothed wheel is a straight line. The height of the openings between the teeth are not important when it comes to defining the distinct positions but can be relevant when it comes to making the connection between the connector part 2 and the site 1 stable as longer openings causing a longer and closer fit between the corresponding positioning means of the site 1 and of the connector part 2 in a vertical direction, will improve the stability of the connection. According to the second embodiment the outer diameter of the toothed wheel is just slightly larger than the central opening 12 for the cannula part 8 but alternatively the outer diameter could be larger, the optimal diameter is to some degree defined by the connector part 2 which is to be used together with the site 1.

Fig. 3B and D shows a third embodiment of a site 1 according to the invention. The third embodiment comprises a toothed wheel with a bell shaped and/or sigmoid cross section. The sigmoid surface of the teeth can be essentially flat as shown in the figures, but said surface can also be more rounded and comprise further curvatures, such as a convex surface.

According to the second and third embodiment of site, the attachment means 4 comprises a circular protrusion on the periphery of a flat circular base of a hard material which is attached to the surface material 3 of the site 1 i.e. the periphery of the flat circular base has a top section with an increased diameter under which a part of the connector part can get hooked and thereby lock the vertical movement of the connector part 2 relative to the site 1.

Fig. 4 shows a second embodiment of a connector part 2 according to the invention which can connect to the second and the third embodiments of the site. The connector part 2 comprises - like the first embodiment - a hard outer shell 13 having an inner cavity, adapted to encompass the site 1. The outer diameter of the connector part 2 is normally slightly wider than the site 1, but might be similar to or smaller than the outer diameter of the site 1. Furthermore, the connector part 2 comprises means for attachment 6 corresponding to attachment means 4 of the site 1 and means for positioning 7 corresponding to positioning means 5 of the site 1.When the corresponding attachment means 6 are activated, the connector part 2 can be attached releasably to the site 1 as the means for attachment 6 correspond to and/or interact with the attachment means 4 of the site 1. The means for positioning 7 corresponding to the positioning means 5 will - when the connector part 2 is attached to the site 1 - provide that the connector part 2 is positioned in a distinct user-defined position relative to the site 1. When combined, the means for positioning 7 of the connector part 2 and the positioning means 5 of the site 1 are adapted to define more than one distinct position for the connector part 2 in relation to the site 1 which positions the user can choose among when the user joins the two parts are together.

The means for positioning 7 of the connector part 2 comprise elongated protrusions extending from the inner surface of the outer shell 13 in a vertical direction which protrusions are adapted to fit into the openings of the toothed wheel constituting the positioning means 5 of the site 1. As shown in the figures, the protrusions are arranged in a circular fashion and are attached to or being a part of the outer shell 13. When the protrusions are arranged in a circular fashion, the protrusions also work as guiding means helping the user to connect the connector part 2 to the site 1. The protrusions may comprise a curved or inclined surface at the end in order to facilitate finding the appropriate, distinct position. In the shown embodiment, the numbers of protrusions extending from the inner surface of the outer shell 13 matches the number of openings in the toothed wheel providing the positioning means 5 of the site 1. Alternatively, the number of protrusions might be smaller than the number of openings in the positioning means 5 but there should be at least one protrusion and at the most the number of protrusion should equal the number of openings in the positioning means 5. A higher number of protrusions may increase or improve the stability of the assembly comprising the combined site 1 and connection part 2.

The means for attachment 6 of the connector part 2 comprise like for the first embodiment actuating means 15, retaining elements 14 and an elastic element 16. The actuating means 15 comprises two arms positioned opposite each other and an elastic element 16 connects the two arms by one end of each arm. Each arm is extended from the point of connection thereby turning each arm into a form for lever. Each arm extension is provided with an inward hook constituting a retaining element 14. Each hook has a portion which upon release is caught under the protruding upper edge of the flat circular base of a hard material of the site 1 and prevents the connector part 2 from moving away in a vertical direction. The extension of the two arms of the actuating means 15 form part of the outer periphery of the connector part 2. When the two arms forming the actuating means 15 are pressed towards each other the two arm extensions will be moved away from each other thereby increasing the distance between the two retaining elements, the elastic element will provide a spring action trying to return the extended ends of the arms to their original relaxed position. According to this embodiment the elastic element 16 might be made from a different material than the arms.

When the distance between the two retaining elements is increased, it is possible to attach or remove the connector part 2 to/from the site 1. When applying pressure to the actuating means 15 the load of the elastic element 16 in is increased. Inwards pressure is released the return of the actuating means 15 to their relaxed state is assisted by the action of an elastic element 16, the retaining elements move back into their original position, whereby the distance between the retaining elements is reduced to a distance smaller than the outer diameter of the protrusion of the attachment means 4.

Fig. 5 shows top and bottom views of a device according to the invention relating to a leakage- and/or malfunction indication system. The four views shows a surface material 3 in the form of a round transparent plaster, in the central part of the plaster is placed a round part of a hard non-transparent material such as a top part to which a connector part 2 can be attached. Around the part of hard non-transparent material a ring-shaped section of the plaster is impregnated with a leak indicator 20 which shifts colour when it gets in contact with leaking medication such as insulin. The two left examples shows top views of the device; the two right examples show bottom views of the same device as shown to the left.

Example A illustrates a "complete crawl out" where the cannula is no longer subcutaneously inserted but is positioned above the skin under the transparent plaster. When a coloured cannula is applied, the cannula becomes visible through the transparent plaster during use and the patient is able to realise that the insulin input is not working correctly although the indication does not change colour.

Example B illustrates a "partial crawl out" or "buckling/crimping" of the cannula. In this case the cannula is not visible through the transparent plaster but it is also not functioning as it should. When insulin reaches the edge of the hard non-transparent part, the leakage indicator will change colour. Thus, the patient will be able to realise that the cannula is malfunctioning and that the insulin input might be incorrect.

The leak indicators might be used for any medication for which it is possible to produce a visible change. The leak indicators can be used with all embodiments of the present invention.

| **Ref. No** | **Part** |
|---|---|
| 1 | Infusion site |
| 2 | Connector part |
| 3 | Surface of material |
| 4 | Attachment means for the connector part |
| 5 | Positioning means |
| 6 | Means for attachment corresponding to means 4 |
| 7 | Means for positioning corresponding to means 5 |
| 8 | Cannula part |
| 9 | Septum |
| 10 | Circular part |
| 11 | Central part |
| 12 | Opening for cannula part |
| 13 | Outer shell |
| 14 | Retaining means |
| 15 | Actuating means |
| 16 | Elastic element |
| 17 | Cylindrical wall |
| 18 | Tubular element |
| 19 | Top part |
| 20 | Leak indicator |

## Claims

1. A medical device constituted by a site (1) and a connector part (2) where the site (1) is stationary during use and attached to a patients skin and the connector part (2) can be attached to the site (1) during use
which site (1) comprises
- a surface material (3) to be attached to the patients skin;
- an inlet for fluid and a fluid path leading the fluid to a subcutaneous position;
- positioning means (5) for the connector part (2) configured to provide a stationary i.e. fixed positioning of the connector part (2) relative to the site (1) in a horizontal direction during use; and
which connector part (2) comprises
- an outlet for fluid (18) which during use protrudes through the inlet for fluid of the site (1);
- means for positioning (7) corresponding to the positioning means (5) on the site (1); **characterized in that** the medical device is a two piece device in the use-situation and the site (1) is provided with positioning means (5) defining more than one possible position for the connector part (2) during use.

2. A medical device according to claim 1, wherein the connector part (2) is releasably attached to the site (1) and the site (1) comprises attachment means (4) for the connector part (2) providing releasable attachment of the connector part (2) to the site (1) during use; and the connector part (2) comprises corresponding means for attachment (6) configured to interact with the attachment means (4) on the site (1).

3. A medical device according to claim 1 or 2, wherein the positioning means (5) of the site (1) comprise at least two openings (5) where at least one opening (5) correspond(s) to a protruding part (7) on the connector part (2) when the connector part (2) is placed in a use position.

4. A medical device according to claim 3, wherein the positioning means (5) of the site (1) comprise at least two openings (5) where two or more openings (5) correspond to two or more protruding part (7) on the connector part (2) when the connector part (2) is placed in a use position.

5. A medical device according to claim 3 or 4, wherein the positioning means (5) of the site (1) comprise 2 to 10 openings (5) corresponding to 2 to 10 protruding parts (7) on the connector part (2) when the connector part (2) is placed in a use position.

6. A medical device according to any preceding claim, wherein the site (1) has a subcutaneous part when positioned on the patients skin or has means for attaching a subcutaneous part after positioning the site on the patients skin.

7. A medical device according to any preceding claim, wherein the attachment means (4) of the site (1) comprises an increase or a decrease in a cross-sectional dimension providing an inward or outward step in a vertical profile.

8. A medical device according to claim 7, wherein an outward rim at an outer vertical surface or an inward rim at an inner vertical surface is provided.

9. A medical device according to any preceding claim, wherein the means for attachment (6) of the connector part (2) comprises actuation means (15) in the form of at least one arm placed along the outer edge of the connector part (2) which arm is fastened to the connector part (2) at one end and can be pivoted around this fastening point, the arm is provided with retaining means (14) configured to prevent vertical movements of the connector part (2) relative to the site (1).

10. A medical device according to any preceding claim, wherein the means for attachment (6) of the connector part (2) comprises actuation means (15) in the form of two oppositely positioned arms which arms each are pivotally attached at one end and each arm is provided with retaining means (14) and the distance between the retaining means on each arm can be varied due to the pivotal movement of the two arms.

11. A medical device according to any preceding claim, wherein the connector part (2) comprises a connector needle in the form of a tubular element (18) to be inserted through a septum of the site (1).

12. A medical device according to claim 11, wherein the connector part (2) is provided with guiding means (17) adapted to guide the tubular element (18) through a penetratable part of the site (1) in a vertical direction.

13. A medical device according to any one of the preceding claims, wherein the device comprises a leakage indication system comprising leakage indication means adapted to provide a colour change upon leakage of a fluid, the indication means being provided on a surface of the site (1) near the insertion site, and being visible i.e. inspectable upon use.

14. A medical device according to claim 13, wherein the leakage indication system comprises a reference marker indicating the visual change to be expected upon leakage.

15. A medical device according to any one of the preceding claims, wherein the device comprises a subcutaneous part comprising a coloured or contrast-enhanced cannula, and the site (1) comprises a transparent area arranged near the insertion site, said transparent area being adapted to allow visible detection of misplacement of the cannula during use.
